(19) Europäisches Patentamt
European Patent Office
Office européen des brevets

(11) EP 1 409 743 B1

(12) FASCICULE DE BREVET EUROPEEN

(45) Date de publication et mention de la délivrance du brevet: **31.05.2006 Bulletin 2006/22**

(21) Numéro de dépôt: **02764983.9**

(22) Date de dépôt: **12.07.2002**

(51) Int Cl.: ***C12Q 1/68*** *(2006.01)* ***C12Q 1/26*** *(2006.01)*

(86) Numéro de dépôt international: **PCT/FR2002/002482**

(87) Numéro de publication internationale: **WO 2003/008641 (30.01.2003 Gazette 2003/05)**

(54) **PROCEDE ET SUPPORT D'ANALYSE BIOLOGIQUE UTILISANT DES OLIGONUCLEOTIDES COMPORTANT UN MARQUEUR ACTIVABLE ENZYMATIQUEMENT**

VERFAHREN UND TRÄGER ZUR BIOLOGISCHEN ANALYSE UNTER VERWENDUNG VON EINEN ENZYMATISCH AKTIVIERBAREN MARKER TRAGENDEN OLIGONUKLEOTIDEN

METHOD AND SUPPORT FOR BIOLOGICAL ANALYSIS USING OLIGONUCLEOTIDES COMPRISING A MARKER CAPABLE OF ENZYMATIC ACTIVATION

(84) Etats contractants désignés:
**AT BE BG CH CY CZ DE DK EE ES FI FR GB GR IE IT LI LU MC NL PT SE SK TR**

(30) Priorité: **16.07.2001 FR 0109460**

(43) Date de publication de la demande: **21.04.2004 Bulletin 2004/17**

(73) Titulaires:
- **COMMISSARIAT A L'ENERGIE ATOMIQUE 75752 Paris Cedex 15 (FR)**
- **UNIVERSITE JOSEPH FOURIER F-38041 Grenoble Cédex (FR)**

(72) Inventeurs:
- **DECOUT, Jean-Luc F-38410 Vaulnaveys-le-Haut (FR)**
- **FONTECAVE, Marc F-38330 Saint-Ismier (FR)**
- **DUEYMES, Cécile F-12000 Rodez (FR)**

(74) Mandataire: **Poulin, Gérard Brevatome 3, rue du Docteur Lancereaux 75008 Paris (FR)**

(56) Documents cités:
**EP-A- 0 097 373**
**EP-A- 0 461 731**
**WO-A-01/63282**
**US-A- 4 959 309**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 409 743 B1

Printed by Jouve, 75001 PARIS (FR)

## Description

### Domaine technique

La présente invention a pour objet un procédé et un support d'analyse destinés à la détermination de cibles biologiques du type ADN ou ARN. Ce support et ce procédé trouvent des applications dans de nombreux domaines, en particulier en biologie pour le séquençage des génomes, la recherche des mutations, le développement de nouveaux médicaments, etc...

### Etat de la technique antérieure

Les procédés et supports d'analyse de ce type utilisent généralement une pluralité de sondes oligonucléotidiques susceptibles de donner lieu à une hybridation avec les cibles biologiques à analyser. L'hybridation correspond à l'appariement des brins-cibles avec les brins d'ADN complémentaires, soit les sondes, disposés à des coordonnées précises sur le support. Pour déterminer la nature des cibles, il est donc nécessaire de pouvoir identifier quels sont les sites du support et donc les oligonucléotides, qui ont donné lieu à une hybridation.

Dans le document US-A-5 925 525 [1], on détermine l'hybridation des cibles biologiques sur le support à l'aide d'un marqueur fluorescent qui est associé aux cibles biologiques. Ainsi, après mise en contact des cibles marquées avec le support d'analyse comportant les oligosondes, puis lavage, on détermine les sites où a eu lieu l'hybridation en réalisant une excitation de l'ensemble des marqueurs fluorescents, suivie d'une localisation des sites par détection de la lumière de fluorescence réémise par les marqueurs. Les sites pour lesquels une lumière de fluorescence est détectée sont ceux qui ont fixé des molécules-cibles.

Cette technique présente l'inconvénient de nécessiter le marquage de la cible biologique avant sa mise en contact avec le support d'analyse, ce qui pose certains problèmes pratiques en ce qui concerne la quantification et/ou le rendement du marquage et le délai pour l'effectuer. De plus, le marquage à une étape donnée fige la situation et empêche de réaliser par la suite des mesures dynamiques capables de suivre une réaction d'hybridation.

Une autre technique pour détecter et identifier un acide nuclétique (cible) par sa capacité d'hybridation, décrite dans US-A-5,482,832 [2], consiste à marquer ces sondes avec une enzyme, puis à mettre en contact les oligonucléotides marqués par une enzyme avec les cibles. La séparation des oligonucléotides non-hybridés des duplex se fait par électrophorèse sur gel. La détection et la détermination de l'hybridation se fait, après migration, en colorant le gel au moyen d'un substrat approprié.

Cette technique sur gel limite le nombre de cibles testées et nécessite donc la fixation d'enzymes sur les sondes oligonucléotidiques, ce qui peut poser certains problèmes et risque, dans certains cas, de perturber l'hybridation de l'oligonucléotide avec l'oligonucléotide complémentaire.

EP-A-0 097 373 décrit des sondes oligonucléotidiques qui sont marquées par un cofacteur et qui sont reconnues par l'enzyme correspondante, par exemple la flavine et la flavine réductase. Ces sondes sont utilisées pour la détection d'acides nucléiques par hybridation.

US-A-4 959 309 décrit des sondes oligonucléotidiques qui sont marquées par un cofacteur et qui sont reconnues par l'enzyme correspondante. Lesdites sondes peuvent être immobilisées. Ces sondes sont utilisées pour la détection d'acides nucléiques par hybridation.

Ces deux documents ne mentionnent pas un changement de signal par rapport à l'hybridation de molécules cibles.

WO 01/63282 décrit un support d'analyse comportant des oligonucléotides fixés qui sont marqués par la flavine ou un de ses dérivés.

Les oligonucléotides sont utilisés pour la détections d'acides nucléiques par hybridation. La fluorescence des oligonucléotides marqués est atténuée après hybridation.

### Exposé de l'invention

La présente invention a précisément pour objet un procédé d'analyse de cibles biologiques utilisant des sondes portant un marqueur activable enzymatiquement, mais qui ne nécessite pas la fixation d'une enzyme sur des sondes oligonucléotidiques.

Selon l'invention, le procédé d'analyse de cibles biologiques de type ADN ou ARN, comprend les étapes suivantes :

a) mettre en contact les cibles à analyser avec des sondes oligonucléotidiques marquées par un cofacteur A d'une enzyme E, le cofacteur étant tel qu'il est reconnu par l'enzyme E lorsqu'il est fixé sur un oligonucléotide libre et qu'il est moins reconnu par l'enzyme E lorsque l'oligonucléotide sur lequel il est fixé, est hybridé avec un oligonucléotide

complémentaire ;

b) ajouter au milieu réactionnel l'enzyme E correspondant au cofacteur A et un substrat S de l'enzyme E, le substrat S étant converti par l'enzyme E en un composé C ;

c) mesurer un signal représentatif de l'activité de l'enzyme E sur le substrat S ; et

d) comparer ce signal avec le signal obtenu lorsqu'on met en contact les sondes oligonucléotidiques marquées par le cofacteur A avec l'enzyme E et le substrat S, dans les mêmes conditions, mais en l'absence des cibles, la différence entre les deux signaux indiquant la présence de cibles complémentaires des sondes oligonucléotidiques.

Dans ce procédé, on tire profit du fait que le cofacteur A est moins reconnu par l'enzyme E lorsque les sondes oligonucléotidiques marquées par le cofacteur A sont hybridées avec des cibles complémentaires. De ce fait, l'activité de l'enzyme E sur le substrat S chute lorsqu'il y a hybridation.

De préférence, pour obtenir une comparaison significative de l'activité enzymatique de l'enzyme E sur le substrat S, le cofacteur A est tel qu'il n'est plus ou presque plus reconnu par l'enzyme E lorsque les sondes oligonucléotidiques marquées par le cofacteur A sont hybridées avec des cibles biologiques complémentaires.

De préférence encore, le signal mesuré dans l'étape c) représente au plus 50% du signal obtenu en l'absence des cibles biologiques.

La présente invention est ainsi basée sur le principe selon lequel on obtient un signal qui varie lors de l'hybridation de l'oligonucléotide marqué avec un oligonucléotide complémentaire. Dans le cas de l'invention, la variation de ce signal est basée sur l'utilisation d'une enzyme E qui déclenche ce signal et sur l'effet amplificateur de l'enzyme sur l'intensité de ce signal, ce qui permet une amélioration du rapport signal/bruit.

Pour mettre en oeuvre le procédé de l'invention, on choisit de préférence un système : cofacteur A, enzyme E et substrat S, tel qu'il permette une détection de la chute de l'activité enzymatique au moyen d'un signal optique.

On peut détecter cette chute d'activité en utilisant par exemple un substrat S présentant des propriétés d'absorption de lumière et/ou de fluorescence spécifiques qui sont différentes des propriétés d'absorption de lumière et/ou de fluorescence du composé C obtenu par transformation du substrat S sous l'action de l'enzyme E.

On pourrait aussi détecter cette chute d'activité enzymatique, dans le cas où le composé C de conversion du substrat présente des propriétés d'absorption de lumière et/ou de fluorescence spécifiques, en mesurant un signal optique représentatif de la quantité de composé C formé.

Ainsi, en l'absence d'oligonucléotide cible complémentaire, l'enzyme E reconnaît le cofacteur A fixé sur la sonde oligonucléotidique et une activité enzymatique peut être mesurée, après mise en contact de la sonde oligonucléotidique marquée par le cofacteur A avec l'enzyme E et le substrat S. La réaction enzymatique correspond au schéma suivant :

$$\text{Substrat S} \xrightarrow[\text{Co-facteur A}]{\text{Enzyme E}} \text{Composé C}$$

Dans le cas où le substrat S présente un maximum d'absorption à une longueur d'onde $\lambda_1$ ou un maximum d'émission de fluorescence à une longueur d'onde $\lambda_2$ pour lesquelles le composé C ne présente pas de propriétés d'absorption de lumière ou d'émission de fluorescence, on peut déterminer l'activité enzymatique par la variation de densité optique ($\Delta$DO) à la longueur d'onde $\lambda_1$ ou par la variation de fluorescence ($\Delta$fluo) à la longueur d'onde $\lambda_2$, en fonction du temps.

Lorsque la sonde oligonucléotidique marquée par le cofacteur A est hybridée avec un oligonucléotide cible complémentaire, l'enzyme E ne reconnaît plus ou presque plus le cofacteur A et le substrat S n'est plus ou presque plus transformé en composé C. Il en résulte que $\Delta$DO/min ou $\Delta$fluo/min diminue.

De préférence, on utilise un système : cofacteur A, enzyme E, substrat S, tel que cette diminution représente de 50 à 100% de la valeur maximale $\Delta$DO/min ou $\Delta$fluo/min obtenue avec la sonde oligonucléotidique marquée par A, seule, c'est-à-dire sans oligonucléotide complémentaire.

La reconnaissance du cofacteur A fixé sur la sonde oligonucléotidique par l'enzyme E varie en fonction du nombre de bases complémentaires présentes sur l'oligonucléotide cible. Il en résulte une relation entre $\Delta$DO/min ou $\Delta$fluo/min en fonction de ce nombre de bases. Ceci permet d'effectuer une analyse fine du degré de complémentarité entre l'oligonucléotide cible à analyser et la sonde oligonucléotidique marquée.

Par ailleurs, la détection des hybridations peut être obtenue en mesurant la variation d'un signal optique (variation de D0 ou de fluorescence) liée à la consommation du substrat S et engendrée par l'action d'une enzyme.

De plus, la consommation de S et l'amplitude du signal peuvent être rendues importantes grâce à l'effet d'amplification dû à l'enzyme.

Selon l'invention, il est également important que le processus de reconnaissance entre le cofacteur A et l'enzyme

E ne soit pas basé sur la formation d'une liaison covalente entre ces deux éléments, c'est-à-dire que le cofacteur A ne s'accroche pas de façon covalente à l'enzyme E, afin que l'on puisse obtenir cet effet d'amplification.

Selon un mode de réalisation du procédé de l'invention, le cofacteur A est la flavine ou l'un de ses dérivés.

Généralement, ces composés sont fixés sur des oligonucléotides par l'intermédiaire d'un bras espaceur, par exemple de type $-(CH_2)_n-$ pour donner des conjugués flavine-oligonucléotide. Une synthèse de conjugués de ce type est décrite par C. Frier et coll. dans J. Org. Chem., 62, 1997, p.3520-3528 [3].

Cette synthèse consiste à fixer un dérivé de flavine sur l'oligonucléotide par une méthode de couplage phosphoramidite ou H-phosphonate en partant d'un dérivé de formule :

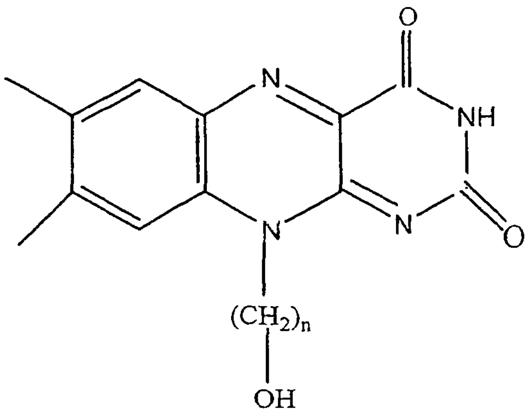

(I)

avec n étant un nombre entier de 2 à 8, par exemple 6.

La stratégie de synthèse permet d'introduire le groupe flavinique, y compris dans des procédés de synthèse oligonucléotidiques automatisés.

Ainsi, l'oligonucléotide est marqué par un groupe de formule $-(CH_2)_n-R^1$ dans laquelle n est un nombre entier allant de 2 à 8, et $R^1$ représente le groupe de formule :

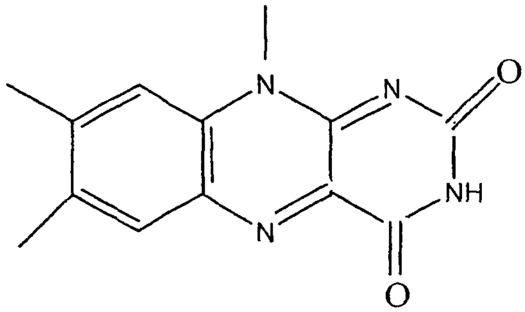

(II)

Les enzymes E susceptibles d'être utilisés avec ce cofacteur, peuvent être des flavoprotéines parmi lesquelles on trouve différentes oxydases. De préférence, dans l'invention on utilise les NAD(P)H oxydases et les NAD(P)H : flavine oxydoréductases encore appelées flavine réductases.

A titre d'exemple de NAD(P)H oxydase, on peut citer celle issue de la levure et appelée Old Yellow Enzyme décrite par J. Fisher et coll., Biochemistry, 1976, 15, p. 1054-1063 [4].

A titre d'exemple de flavine réductase, on peut citer celle décrite par F. Fieschi et coll. J. Biol. Chem., 1995, 270, p. 30392-30400 [5].

Les substrats utilisables avec ces enzymes peuvent être le NADPH et le NADH.

L'utilisation des flavines comme cofacteur est très intéressante car ce sont des molécules qui agissent comme des cofacteurs des flavine réductases, c'est-à-dire des enzymes solubles qui catalysent l'oxydation du NADPH ou du NADH par l'oxygène de l'air selon le schéma suivant :

$$\text{NAD(P)H} \xrightarrow[\text{Flavine, } O_2]{\text{Flavine réductase}} \text{NAD(P)}^+$$

fluorescent à $\lambda_2$=460 nm — non fluorescent

absorption maximale à $\lambda_1$=340 nm — pas d'absorption

Dans ce schéma, la flavine réductase catalyse la réduction des flavines par le NAD(P)H. Les flavines réduites produites par la réaction réagissent ensuite spontanément avec l'oxygène de l'air pour reformer les flavines à l'état initial. De nombreux cycles de transformation peuvent être ainsi effectués permettant une consommation importante de NADPH ou NADH. Ce phénomène d'amplification enzymatique permet l'obtention de signaux importants.

Dans l'invention, l'emploi du NADPH ou du NADH comme substrat, est particulièrement intéressant car ces molécules sont douées d'une absorption de lumière et d'une émission de fluorescence caractéristiques que ne possèdent pas leurs produits d'oxydation $NADP^+$ et $NAD^+$.

Les propriétés d'émission de fluorescence du NADH sont décrites en particulier par Scott et coll. dans J. Am. Chem. Soc., 92:3, 11 février 1970, p. 687 à 695 [6].

Ainsi, le NADH présente une fluorescence maximale à la longueur d'onde de 460 nm, et une absorption maximale à la longueur d'onde de 340 nm alors que son produit de conversion par l'enzyme $NAD^+$ ne présente pas de fluorescence et pas d'absorption.

Par ailleurs, la flavine oxydoréductase constitue un excellent exemple d'enzyme utilisable dans l'invention. En effet, il s'agit d'une protéine monomérique, soluble, stable, facile à purifier à partir d'une souche surproductrice. La préparation de cette enzyme est décrite dans la référence [5]. Cette enzyme reconnaît exclusivement le cycle isoalloxazine de la flavine et peut donc utiliser comme cofacteurs une très grande gamme d'analogues de flavines présentant des modifications sur la chaîne ribityle, tel que décrit dans la référence [5].

Selon l'invention, on a découvert que le groupement flavinique fixé sur l'oligonucléotide était reconnu par la flavine réductase de sorte que le conjugué oligonucléotide-flavine constitue toujours un cofacteur permettant à l'enzyme de catalyser l'oxydation du NADPH ou du NADH et donc de déclencher une diminution de l'absorption de lumière à 340 nm et une diminution de la fluorescence à 460 nm.

Lorsque le cofacteur flavinique A n'est pas en condition saturante par rapport à la concentration de l'enzyme E, la variation de DO en fluorescence ($\Delta$DO ou $\Delta$fluo) par unité de temps est proportionnelle à la quantité de cofacteur flavinique A. Ceci permet une quantification des sondes marquées par le cofacteur A.

Selon l'invention, on a découvert que lorsqu'un conjugué oligonucléotide-flavine est hybridé à un oligonucléotide complémentaire, le groupement flavinique n'est plus reconnu par la flavine réductase qui de ce fait n'est plus capable de catalyser l'oxydation du NADPH ou du NADH et de déclencher la diminution d'absorption à 340 nm et de fluorescence à 460 nm.

En conclusion, sans hybridation, la sonde oligonucléotide-flavine (oligo-A) est détectée par une mesure de signal consistant en une diminution de la densité optique ou de la fluorescence par unité de temps ou pour un temps fixé, par exemple 5 minutes. Ce signal tend vers une extinction partielle ou totale ($\Delta$DO ou $\Delta$fluo diminue ou dans le meilleur des cas devient nul) et ce spécifiquement quand il y a hybridation avec une cible complémentaire non marquée. La preuve de l'hybridation peut donc être détectée par la variation de la diminution de densité optique ou de l'émission de fluorescence.

Un avantage de ce procédé est que cette variation du signal mesuré n'est pas le reflet de la variation de la densité optique ou de la fluorescence d'une molécule donnée mais de plusieurs molécules de substrat S de l'enzyme E utilisant A comme cofacteur. La réaction enzymatique permet donc une amplification considérable de la réponse.

Selon une variante de réalisation de l'invention, utilisable dans le cas du système : flavine (cofacteur A), flavine réductase (enzyme E) et NADH ou NADPH (substrat), on peut obtenir un signal représentatif de l'activité enzymatique de l'enzyme E en utilisant une seconde enzyme et un aldéhyde, la seconde enzyme étant capable de catalyser la réaction de la flavine réduite avec l'aldéhyde et l'oxygène, cette réaction étant accompagnée d'une luminescence représentative de l'activité de l'enzyme E. La seconde enzyme est par exemple la luciférase, et l'aldéhyde peut être le décanal.

Dans un premier temps, comme il est décrit précédemment, le substrat S est converti en composé C par la première enzyme E (flavine réductase) en formant de la flavine réduite. Dans un deuxième temps, la seconde enzyme (la luciférase) catalyse la réaction de la flavine réduite avec l'aldéhyde (RCHO) et l'oxygène, selon le schéma réactionnel suivant : Flavine réduite+RCHO+$O_2 \rightarrow$ RCOOH+flavine oxydée+ (lumière) (hv)

Ceci correspond à une luminescence à 490 nm.

Cette réaction est décrite en particulier par Hastings et coll., Advances in Microbial Physiology, vol. 26, 1985, p. 235-291 [7] et par Zenno et coll., J. Bacteriol., 1994, p. 3536-3543 [8].

La flavine oxydée ainsi formée peut être utilisée par la flavine réductase pour convertir le substrat S et être à

nouveau réduite.

Ainsi, lorsque la sonde est hybridée avec une cible complémentaire, ces réactions ne se produisent plus et il n'y a plus de lumière. Avec l'oligoflavine, cela donne donc de la lumière quand il n'y a pas d'hybridation et n'en donne plus quand il y a hybridation.

La détection de l'hybridation par des mesures de luminescence est intéressante car le signal est spécifiquement lié à une réaction chimique et pas aux propriétés intrinsèques (absorption ou fluorescence) des objets (moyens) utilisés.

On a donc quelque chose de très spécifique car les supports peuvent être un peu fluorescents et cela peut affecter les mesures de fluorescence. En revanche, ils ne sont pas chimioluminescents.

Dans cette variante de l'invention, on peut utiliser la flavine réductase de E. coli ou de V. harveyi, et la luciférase de V. harveyi.

Dans ce cas, on ajoute à la sonde marquée à la flavine, la flavine réductase et la luciférase, du décanal, par exemple 0,001% vol/vol, du NADPH ou du NADH 200 $\mu$M, du tampon phosphate, pH 7,50 mM. On mesure la luminescence avec un lumiphotomètre commercial à 490 nm. Lorsqu'on répète cette opération avec un oligonucléotide cible complémentaire, il n'y a pas de luminescence.

Selon l'invention, les sondes oligonucléotidiques marquées par le cofacteur A sont de préférence fixées sur un support solide. Ce support solide peut être muni de microcuvettes dans lesquelles sont fixées les sondes.

Aussi décrit dans la demande est un support d'analyse de cibles biologiques de type ADN ou ARN comportant des sondes oligonucléotidiques fixées sur un support solide, caractérisé en ce que les sondes oligonucléotidiques sont marquées par un cofacteur A d'une enzyme E tel que le cofacteur A fixé sur la sonde est reconnu par l'enzyme E, mais qu'il est moins reconnu par l'enzyme E lorsque la sonde oligonucléotidique est hybridée avec un oligonucléotide complémentaire, l'enzyme E étant telle qu'elle possède un substrat S ou un composé C de conversion de ce substrat qui permet de déterminer l'activité de l'enzyme E par un signal optique, représentatif de la consommation du substrat S ou de la production du composé C.

De préférence, le cofacteur A est la flavine ou l'un de ses dérivés.

Ainsi, la sonde oligonucléotidique peut être marquée par un groupe de formule $-(CH_2)_n-R^1$ dans laquelle n est nombre entier allant de 2 à 8 et $R^1$ représente un groupe répondant à la formule suivante :

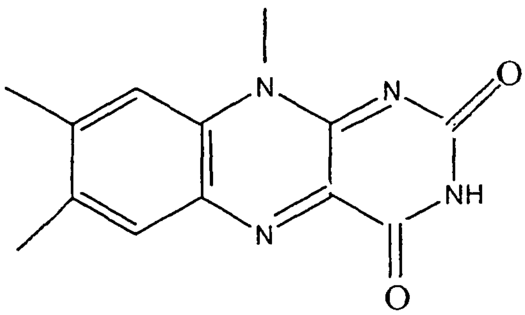

(II)

**Brève description des dessins**

La figure 1 est une courbe illustrant l'évolution de l'activité enzymatique de la flavine réductase en fonction de quantités croissantes d'un oligonucléotide cible complémentaire.

La figure 2 illustre l'activité enzymatique déterminée en fonction du nombre de bases complémentaires de l'oligonucléotide-cible.

La figure 3 représente le spectre d'émission de fluorescence du NADH.

La figure 4 illustre la variation de l'intensité de fluorescence du NADH en présence de la flavine réductase et de la sonde oligonucléotique seule (droite ISIS), ou hybridée à un oligonucléotide cible complémentaire (droite ISIS + ICAM-20).

La figure 5 illustre l'effet du nombre de bases complémentaires d'un oligonucléotide cible hybridé à l'oligonucléotide sonde marqué par la flavine, sur la variation de l'intensité de fluorescence du NADH induite par l'activité de la flavine réductase.

La figure 6 illustre l'activité enzymatique de la flavine réductase en fonction de quantités croissantes de sondes marquées à la flavine.

## Exposé détaillé des modes de réalisation

Dans les exemples qui suivent, on utilise les oligonucléotides suivants :

1) sonde oligonucléotide-flavine
ISIS : SEQ ID N°: 1
3'-ctctcccctt caccaccccc -p-$C_6$flavine
2) cibles complémentaires de la sonde oligonucléotide-flavine
ICAM-20 : SEQ ID N° : 2
5'-gcctgatgag aggggaagtg gtgggggaga catagcccca cc-3'
ICAM-17 : SEQ ID N° . 3
5'-gcctgatgag aggggaagtg gtggcccaga catagcccca cc-3'
ICAM-14 : SEQ ID N° : 4
5'-gcctgatgag aggggaagtg gattcccaga catagcccca cc-3'
3) oligonucléotide non complémentaire
NC : SEQ ID N° : 5
5'-ctcatcgtgt aaaaaaaaaa aggcagtact ggaagggcta attct-3'

La sonde oligonucléotide-flavine ISIS a été synthétisée selon la méthode décrite dans la référence [6].

Dans ces exemples, on utilise comme enzyme E, la flavine réductase préparée selon la méthode décrite dans la référence [8].

Les spectres d'émission de fluorescence et d'excitation ont été enregistrés sur un spectrofluorimètre LS 50B Perkin-Elmer équipé d'une source au xénon pulsé, d'un monochromateur d'excitation, d'un monochromateur d'émission et d'un système de régulation de température.

### Exemple 1

Dans cet exemple, on étudie l'évolution de l'activité enzymatique de la flavine réductase sur la sonde ISIS (SEQ. ID : N°1) en fonction de quantités croissantes d'oligonucléotide-cible complémentaire ICAM-20 (SEQ. ID N° : 2).

On introduit dans la cuve d'un spectrophotomètre Varian-Cary équipé d'un régulateur de température, un volume total de 100 μl comprenant les composés suivants : tampon Tris HCl 50 mM, pH 7,5, NaCl 10 mM, sonde ISIS 10 μM, NADH 200 μM et flavine réductase 0,1 μM ainsi que des quantités croissantes de l'oligonucléotide-cible ICAM-20.

Dans un premier temps, la double hélice entre ISIS et ICAM-20 est formée dans le tampon Tris en présence de NaCl, à température ambiante, et le NADH est ensuite ajouté. La réaction est ensuite déclenchée par ajout de 0,1 μM de flavine réductase (0,3 μg par essai). Elle est suivie pendant quelques minutes à l'air et à 25°C.

On suit l'oxydation du NADH par spectroscopie électronique à 340 nM, longueur d'onde d'absorption caractéristique du NADH.

La figure 1 illustre l'évolution de l'activité enzymatique de la flavine réductase (en nmol NADH oxydé min/mg d'enzyme) en fonction du nombre d'équivalents de la cible ICAM-20 présents.

Les résultats obtenus sur la figure 1 montrent que l'activité enzymatique décroît rapidement lorsque le nombre d'équivalents de la cible ICAM-20 augmente.

### Exemple 2

Dans cet exemple, on vérifie l'influence du nombre de bases complémentaires de la cible sur l'activité enzymatique mesurée.

Dans cet exemple, on suit le même mode opératoire que dans l'exemple 1 mais on utilise de plus, les autres oligonucléotides cibles constitués par ICAM-17 (SEQ. ID N° : 3), ICAM-14 (SEQ. ID N° : 4) et NC (SEQ. ID N° : 5). On utilise dans chaque cas dix équivalents des oligonucléotides cibles.

L'activité enzymatique est déterminée comme dans l'exemple 1.

Les résultats obtenus sont donnés dans le tableau 1 et la figure 2.

**Tableau 1**

| | Activité enzymatique nmol de NADH oxydé/min |
|---|---|
| Cible ICAM-20 : 20 bases complémentaires (SEQ. ID N°: 2 | 200 |
| Cible ICAM-17 : 17 bases complémentaires (SEQ. ID N°: 3) | 500 |

Suite de tableau

| | Activité enzymatique nmol de NADH oxydé/min |
|---|---|
| Cible ICAM-14 : 14 bases complémentaires (SEQ. ID N°: 4) | 1700 |
| Cible non complémentaire (SEQ. ID N°: 5) | 5000 |

La figure 2 et les résultats du tableau I montrent que l'activité enzymatique (en nmol de NADH oxydé/min) diminue fortement à mesure que le nombre de bases complémentaires de l'oligonucléotide cible augmente puisqu'elle passe de 1700 à 200 nmol de NADH oxydé/min lorsqu'on passe de 14 à 20 bases complémentaires.

Dans le cas où la sonde oligonucléotidique est utilisée seule, on obtient une activité enzymatique de 5 000 à 5 800 nmol/min.

**Exemple 3**

Dans cet exemple, on détermine la variation de l'intensité de fluorescence du NADH en présence de la flavine réductase et de la sonde ISIS seule marquée à la flavine, et en présence de la sonde ISIS marquée à la flavine hybridée à l'oligonucléotide complémentaire ICAM-20.

La figure 3 illustre le spectre d'émission de fluorescence du NADH pour une excitation à 340 nm. Sur cette figure, on remarque que le maximum d'émission est à 460 nm.

Dans cet exemple, on utilise un volume total de 400 μl contenant du tampon Tris/HCl, pH 7,6, 50 mM, NaCl 10 mM, la sonde ISIS marquée à la flavine 5 μM, du NADH 200 μM, en présence ou non de la cible complémentaire ICAM-20, 50 μM, 10 équivalents.

La sonde ISIS est mise en solution seule ou appariée avec ICAM-20. Le NADH est ensuite ajouté, puis la réaction est déclenchée à 25°C par ajout de la flavine réductase (0,3 μg par essai), soit 0,1 μM.

L'intensité d'émission de fluorescence (I) du NADH est enregistrée en fonction du temps t (en s) à 460 nm (longueur d'onde maximale d'émission du NADH).

Les résultats obtenus sont donnés sur la figure 4.

Sur cette figure, la droite (ISIS) correspond à la variation de l'intensité de fluorescence pour la sonde ISIS seule, la droite (ISIS + ICAM-20) illustre la variation de l'intensité de fluorescence pour la sonde ISIS hybridée à ICAM-20.

On remarque sur la figure 4 que l'intensité d'émission de fluorescence de la sonde hybridée ne varie pratiquement pas en fonction du temps.

**Exemple 4**

Dans cet exemple, on détermine la variation de l'intensité de fluorescence du NADH en fonction du nombre de paires de bases de la cible complémentaire. On suit le même mode opératoire que dans l'exemple précédent en utilisant soit la cible ICAM-20, soit la cible ICAM-17, soit la cible ICAM-14 à raison de 10 équivalents de cible par rapport à la sonde ISIS. L'intensité d'émission de fluorescence I du NADH exprimée en unité arbitraire est enregistrée à 460 nm pour une longueur d'onde d'excitation de 340 nm, en présence de flavine réductase en fonction du temps. Les droites I=f(t) obtenues permettent de déterminer la variation de fluorescence par minute en fonction du nombre de paires de bases associées dans le duplex formé. Le tableau 2 illustre la variation d'intensité d'émission de fluorescence et la figure 5 illustre cette variation en fonction du nombre de bases complémentaires de l'oligonucléotide cible. Sur cette figure, la valeur 100 de l'unité arbitraire représente la variation de l'intensité de fluorescence par unité de temps dans le cas ISIS non hybridée.

**Tableau 2**

| Cible | Intensité d'émission de fluorescence (unité arbitraire) | | |
|---|---|---|---|
| | I(t=0) | I(t=5 min) | Activité enzymatique |
| Sans | 828 | 688 | 100% |
| ICAM-20 | 828 | 814 | 10% |
| ICAM-17 | 828 | 809 | 20% |
| ICAM-14 | 828 | 803 | 30% |

Si l'on compare les résultats obtenus sur la figure 5 avec ceux de la figure 2 où il s'agissait d'une mesure de densité optique, on voit que l'on obtient des résultats similaires.

**Exemple 5**

Dans cet exemple, on détermine l'activité enzymatique de la flavine réductase, en utilisant des concentrations croissantes de sonde ISIS marquée à la flavine. On réalise l'expérience en l'absence d'oligonucléotides complémentaires, en utilisant une solution de tampon Tris 50 mM, pH 7,6, NaCl 50 mM, NADH 200 μM et 0,05 g/l de flavine réductase. On utilise des quantités de sonde ISIS allant de 0 à 7 μM.

La figure 6 illustre l'activité enzymatique, soit le nombre de nanomoles de NADH oxydé par minute, en fonction de la concentration (en μM) en sonde ISIS. On remarque ainsi que l'activité enzymatique augmente avec la concentration en sonde ISIS, c'est-à-dire en flavine.

**REFERENCES CITEES**

**[1]** US-A-5 925 525,
**[2]** US-A-5,482,832,
**[3]** C. Frier et coll., J. Org. Chem., 62, 1997, p.3520-3528,
**[4]** J. Fisher et coll., Biochemistry, 1976, 15, p. 1054-1063,
**[5]** F. Fieschi et coll. J. Biol. Chem., 1995, 270, p. 20392-20400,
**[6]** Scott et coll., J. Am. Chem. Soc., 92:3, 11 février 1970, p. 687 à 695
**[7]** Hastings et coll., Advances in Microbial Physiology, vol. 26, 1085, p. 235-291
**[8]** Zenno et coll., J. Bacteriol., 1994, p. 3536-3543.

## Revendications

**1.** Procédé d'analyse de cibles biologiques de type ADN ou ARN, qui comprend les étapes suivantes :

a) mettre en contact les cibles à analyser avec des sondes oligonucléotidiques marquées par un cofacteur A d'une enzyme E, le cofacteur étant tel qu'il est reconnu par l'enzyme E lorsqu'il est fixé sur un oligonucléotide libre et qu'il est moins reconnu par l'enzyme E lorsque l'oligonucléotide sur lequel il est fixé, est hybridé avec un oligonucléotide complémentaire ;
b) ajouter au milieu réactionnel l'enzyme E correspondant au cofacteur A et un substrat S de l'enzyme E, le substrat S étant converti par l'enzyme E en un composé C ;
c) mesurer un signal représentatif de l'activité de l'enzyme E sur le substrat S ; et
d) comparer ce signal avec le signal obtenu lorsqu'on met en contact les sondes oligonucléotidiques marquées par le cofacteur A avec l'enzyme E et le substrat S, dans les mêmes conditions, mais en l'absence des cibles, la différence entre les deux signaux indiquant la présence de cibles complémentaires des sondes oligonucléotidiques.

2. Procédé selon la revendication 1, dans lequel le cofacteur A n'est plus ou presque plus reconnu par l'enzyme E lorsque les sondes oligonucléotidiques marquées par le cofacteur A sont hybridées avec des cibles biologiques complémentaires.

3. Procédé selon la revendication 2, dans lequel le signal mesuré dans l'étape c) représente au plus 50% du signal obtenu en l'absence des cibles biologiques.

4. Procédé selon l'une quelconque des revendications 1 à 3, dans lequel le signal représentatif de l'activité enzymatique de l'enzyme E sur le substrat S est un signal optique.

5. Procédé selon l'une quelconque des revendications 1 à 4, dans lequel l'enzyme E et le cofacteur A sont tels que le cofacteur A ne se fixe pas sur l'enzyme E par liaison covalente.

6. Procédé selon l'une quelconque des revendications 1 à 5, dans lequel l'enzyme E est telle que son substrat S ou le composé C de conversion de ce substrat S permet de déterminer l'activité enzymatique de l'enzyme E par une variation de propriétés d'absorption de lumière ou de fluorescence due à la consommation du substrat S ou à la production du composé C.

7. Procédé selon la revendication 5, dans lequel le cofacteur A est la flavine ou l'un de ses dérivés.

8. Procédé selon la revendication 7, dans lequel l'enzyme est une flavoprotéine.

9. Procédé selon la revendication 8, dans lequel l'enzyme E est une flavine réductase ou une NAD(P)H oxydase et le substrat S est le NADPH ou le NADH.

10. Procédé selon la revendication 9, dans lequel le signal mesuré est l'intensité de fluorescence du NADH à 460 nm ou l'absorption de lumière du NADH à 340 nm.

11. Procédé selon la revendication 1, dans lequel le cofacteur A étant la flavine, l'enzyme E étant une flavine réductase et le substrat S étant le NADH ou le NADPH, on obtient le signal représentatif de l'activité de l'enzyme E en utilisant une seconde enzyme et un aldéhyde, la seconde enzyme étant capable de catalyser la réaction de là flavine réduite avec l'aldéhyde et l'oxygène, cette réaction étant accompagnée d'une luminescence représentative de l'activité de l'ensyme E.

12. Procédé selon la revendication 11, dans lequel la seconde enzyme est la luciférase.

13. Procédé selon l'une quelconque des revendications 1 à 12, dans lequel la comparaison des deux signaux permet de déterminer le degré de complémentarité entre la cible et la sonde oligonucléotidique marquée par le cofacteur A.

14. Procédé selon l'une quelconque des revendications 1 à 13, dans lequel on utilise des quantités de substrat telles que l'on amplifie le signal représentatif de l'activité de l'enzyme E.

15. Procédé selon l'une quelconque des revendications 1 à 14, dans lequel les sondes oligonucléotidiques marquées par le cofacteur A sont fixées sur un support solide.

## Claims

1. Method for analyzing biological targets of DNA or RNA type, which comprises the following steps:

   a) contacting the targets to be analyzed with oligonucleotide probes labeled with a cofactor A of an enzyme E, the cofactor being such that it is recognized by enzyme E when it is fixed onto a free oligonucleotide and is less recognized by enzyme E when the oligonucleotide on which it is fixed is hybridized with a complementary oligonucleotide;
   b) adding, to the reaction medium, enzyme E corresponding to cofactor A and a substrate S of enzyme E, substrate S being converted by enzyme E into a compound C;
   c) measuring a signal representative of the activity of enzyme E on substrate S; and
   d) comparing this signal with the signal obtained when the oligonucleotide probes, labeled with cofactor A, are

contacted with enzyme E and substrate S, under the same conditions, but in the absence of the targets, the difference between the two signals indicating the presence of complementary targets of the oligonucleotide probes.

**2.** Method as in claim 1, wherein cofactor A is no longer or almost no longer recognized by enzyme E when the oligonucleotide probes labeled with cofactor A are hybridized with complementary biological targets.

**3.** Method as in claim 2, wherein the signal measured in step c) represents no more than 50% of the signal obtained in the absence of biological targets.

**4.** Method as in any of claims 1 to 3, wherein the signal representative of the enzymatic activity of enzyme E on substrate S is an optic signal.

**5.** Method as in any of claims 1 to 4, wherein enzyme E and cofactor A are such that cofactor A does not fix itself onto enzyme E by covalent bond.

**6.** Method as in any of claims 1 to 5, wherein enzyme E is such that its substrate S or conversion compound C of this substrate S is used to determine the enzymatic activity of enzyme E by a variation in the light absorbing or fluorescence properties due to consumption of substrate S or production of compound C.

**7.** Method as in claim 5, wherein cofactor A is flavin or one of its derivatives.

**8.** Method as in claim 7, wherein the enzyme is a flavoprotein.

**9.** Method as in claim 8, wherein enzyme E is a flavin reductase or a NAD(P)H oxydase and substrate S is NADPH or NADH.

**10.** Method as in claim 9, wherein the signal measured is the intensity of NADH fluorescence at 460 nm or the light absorption of NADH at 340 nm.

**11.** Method as in claim 1, wherein cofactor A being flavin, enzyme E being a flavin reductase and substrate S being NADH or NADPH, the signal representative of the activity of enzyme E is obtained by using a second enzyme and an aldehyde, the second enzyme being able to catalyze the reaction of the flavin reduced with the aldehyde and oxygen, this reaction being accompanied by luminescence representative of the activity of enzyme E.

**12.** Method as in claim 11, wherein the second enzyme is luciferase.

**13.** Method as in any of claims 1 to 12, wherein the comparison of the two signals enables determination of the extent of complementarity between the target and the oligonucleotide probe labeled with cofactor A.

**14.** Method as in any of claims 1 to 13, wherein the quantities of substrate used are such that the signal representative of the activity of enzyme E is amplified.

**15.** Method as in any of claims 1 to 14, wherein the oligonucleotide probes labeled with cofactor A are fixed onto a solid support.

**Patentansprüche**

**1.** Verfahren zum Analysieren von biologischen Targets vom DNA- oder RNA-Typ, das die folgenden Stufen umfasst:

a) Inkontaktbringen der zu analysierenden Targets mit Oligonucleotid-Sonden, die durch einen Cofaktor A eines Enzyms E markiert sind, wobei der Cofaktor ein solcher ist, der von dem Enzym E erkannt wird, wenn er an einem freien Oligonucleotid fixiert ist, und der von dem Enzym E weniger erkannt wird, wenn das Oligonucleotid, an dem er fixiert ist, mit einem komplementären Oligonucleotid hybridisiert ist;
b) Zugabe des Enzyms E, das dem Cofaktor A entspricht, und eines Substrats S für das Enzym E zu dem Reaktionsmedium, wobei das Substrat S durch das Enzym E in eine Verbindung C umgewandelt wird;
c) Messung eines Signals, das repräsentativ ist für die Aktivität des Enzyms E auf dem Substrat S; und

d) Vergleich dieses Signals mit dem Signal, das erhalten wird, wenn man die Oligonucleotid-Sonden, die durch den Cofaktor A markiert sind, mit dem Enzym E und dem Substrat S unter den gleichen Bedingungen, jedoch in Abwesenheit der Targets, in Kontakt bringt, wobei die Differenz zwischen den beiden Signalen die Anwesenheit von Targets anzeigt, die komplementär zu den Oligonucleotid-Sonden sind.

**2.** Verfahren nach Anspruch 1, bei dem der Cofaktor A nicht mehr oder fast nicht mehr erkannt wird von dem Enzym E, wenn die mit dem Cofaktor A markierten Oligonucleotid-Sonden mit komplementären biologischen Targets hybridisiert sind.

**3.** Verfahren nach Anspruch 2, bei dem das in der Stufe (c) gemessene Signal höchstens 50 % des Signals repräsentiert, das in Abwesenheit der biologischen Targets erhalten wird.

**4.** Verfahren nach einem der Ansprüche 1 bis 3, bei dem das Signal, das repräsentativ für die enzymatische Aktivität des Enzyms E auf dem Substrat S ist, ein optisches Signal darstellt.

**5.** Verfahren nach einem der Ansprüche 1 bis 4, bei dem das Enzym E und der Cofaktor A solche sind, dass der Cofaktor A nicht durch covalente Bindung an dem Enzym E fixiert wird.

**6.** Verfahren nach einem der Ansprüche 1 bis 5, bei dem das Enzym E ein solches ist, dass sein Substrat S oder die Umwandlungsverbindung C dieses Substrats S die Bestimmung der enzymatischen Aktivität des Enzyms E durch eine Änderung der Lichtabsorptionseigenschaften oder der Fluoreszenzeigenschaften ermöglicht, die zurückzuführen ist auf den Verbrauch des Substrats S oder auf die Bildung der Verbindung C.

**7.** Verfahren nach Anspruch 5, bei dem der Cofaktor A Flavin oder eines seiner Derivate darstellt.

**8.** Verfahren nach Anspruch 7, bei dem das Enzym ein FlavoProtein darstellt.

**9.** Verfahren nach Anspruch 8, bei dem das Enzym E eine Flavin-Reduktase oder eine DNA (P) H-Oxydase ist und das Substrat S DNAPH oder oder DNAH darstellt.

**10.** Verfahren nach Anspruch 9, bei dem das gemessene Signal die Intensität der Fluoreszenz von DNAH bei 460 nm oder die Lichtabsorption von DNAH bei 340 nm darstellt.

**11.** Verfahren nach Anspruch 1, bei dem man dann, wenn der Cofaktor A Flavin darstellt, das Enzym E eine Flavin-Rduktase darstellt und das Substrat S DNAH oder DNAPH darstellt, das für die Aktivität des Enzyms E repräsentative Signal erhält durch Verwendung eines zweiten Enzyms und eines Aldehyds, wobei das zweite Enzym die Reaktion von reduziertem Flavin mit Aldehyd und Sauerstoff katalysieren kann, die von einer Lumineszenz begleitet ist, die repräsentativ für die Aktivität des Enzyms E ist.

**12.** Verfahren nach Anspruch 11, bei dem das zweite Enzym die Luciferase ist.

**13.** Verfahren nach einem der Ansprüche 1 bis 12, bei dem der Vergleich zwischen den beiden Signalen die Bestimmung des Grades der Komplementarität zwischen dem Target und der mit dem Cofaktor A markierten Oligonucleotid-Sonde erlaubt.

**14.** Verfahren nach einem der Ansprüche 1 bis 13, in dem man solche Mengen an Substrat verwendet, dass das Signal, das für die Aktivität des Enzyms E repräsentativ ist, verstärkt wird.

**15.** Verfahren nach einem der Ansprüche 1 bis 14, in dem die durch den Cofaktor A markierten Oligonucleotid-Sonden an einem festen Träger fixiert sind.

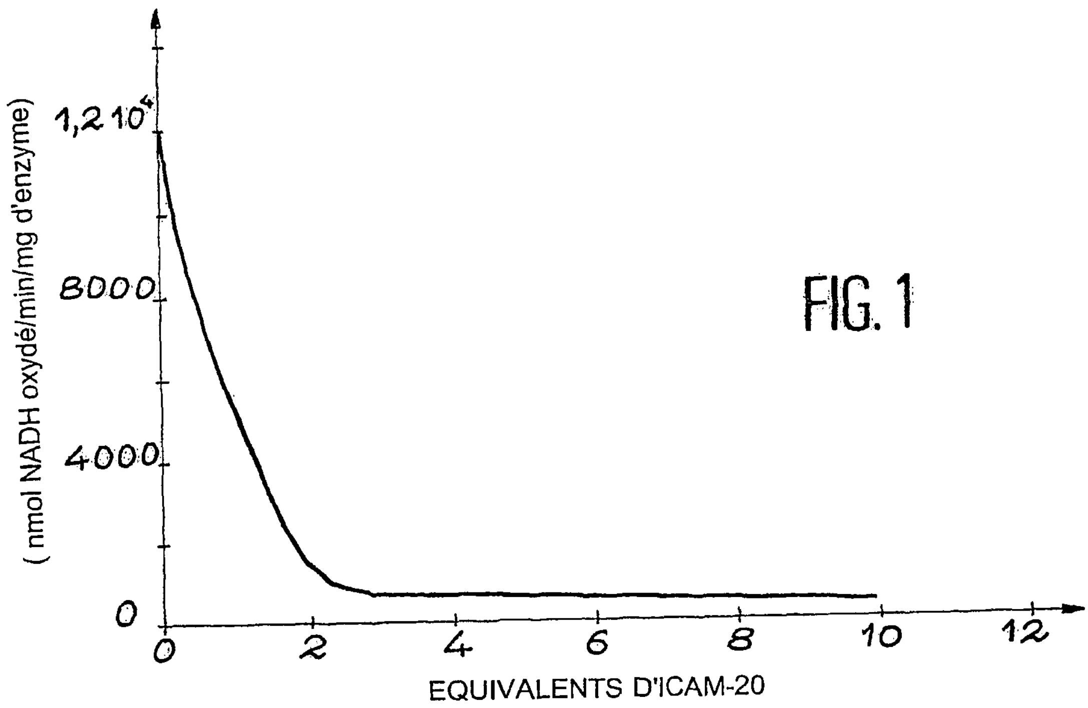
( nmol NADH oxydé/min/mg d'enzyme)
1,2 10⁴
8000
4000
0
0
2
4
6
8
10
12
EQUIVALENTS D'ICAM-20

FIG. 1

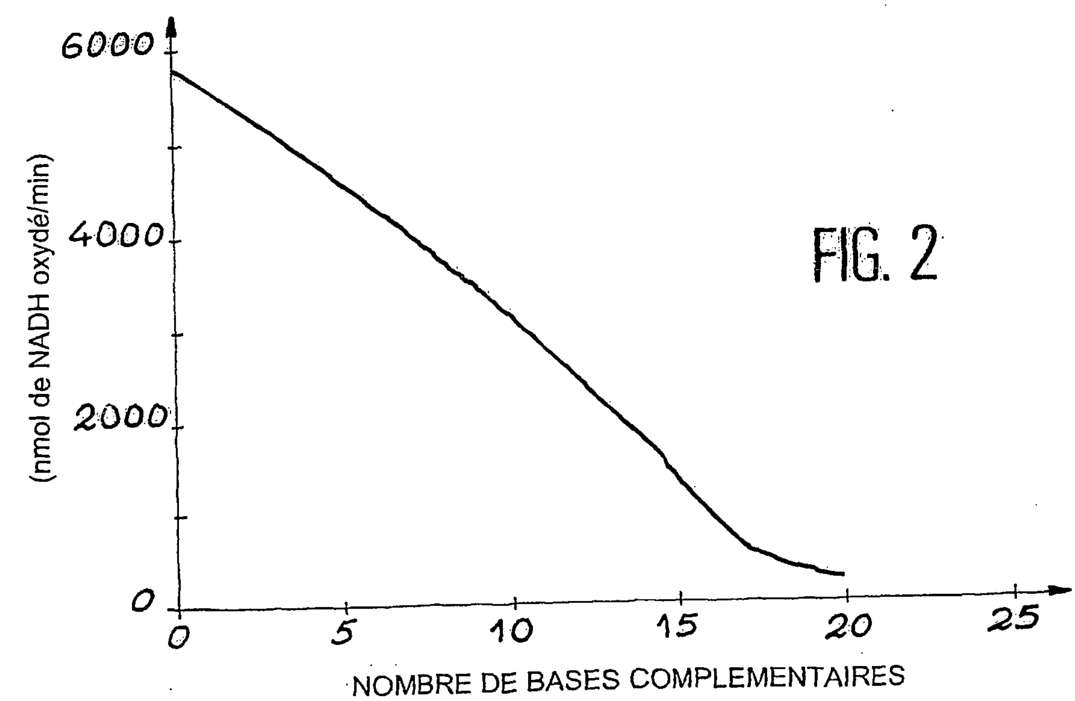
(nmol de NADH oxydé/min)
6000
4000
2000
0
0
5
10
15
20
25
NOMBRE DE BASES COMPLEMENTAIRES

FIG. 2

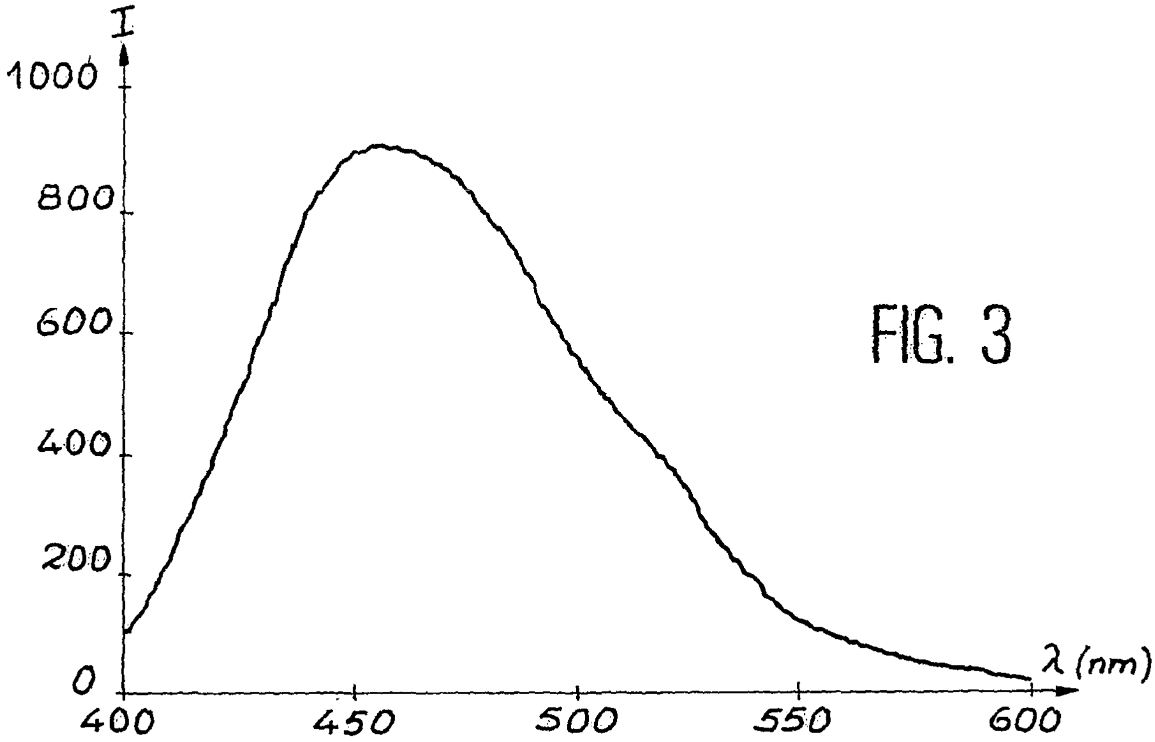
I
1000
800
600
400
200
0
400
450
500
550
600
λ (nm)

FIG. 3

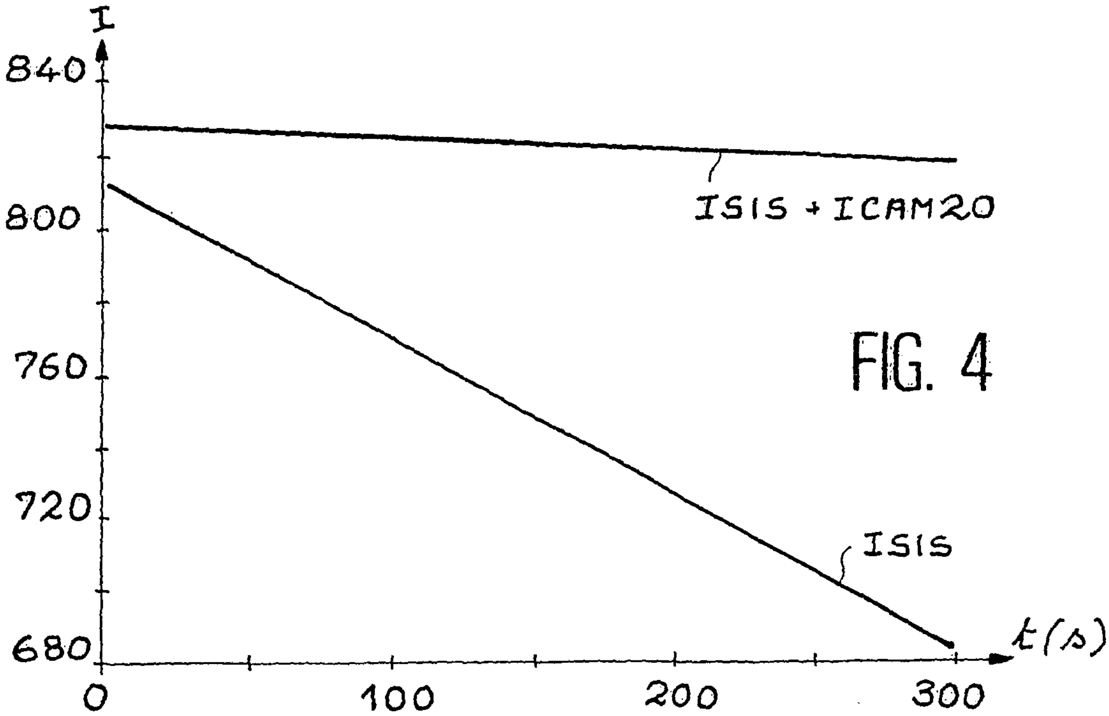
I
840
800
760
720
680
0
100
200
300
t (s)
ISIS + ICAM20
ISIS

FIG. 4

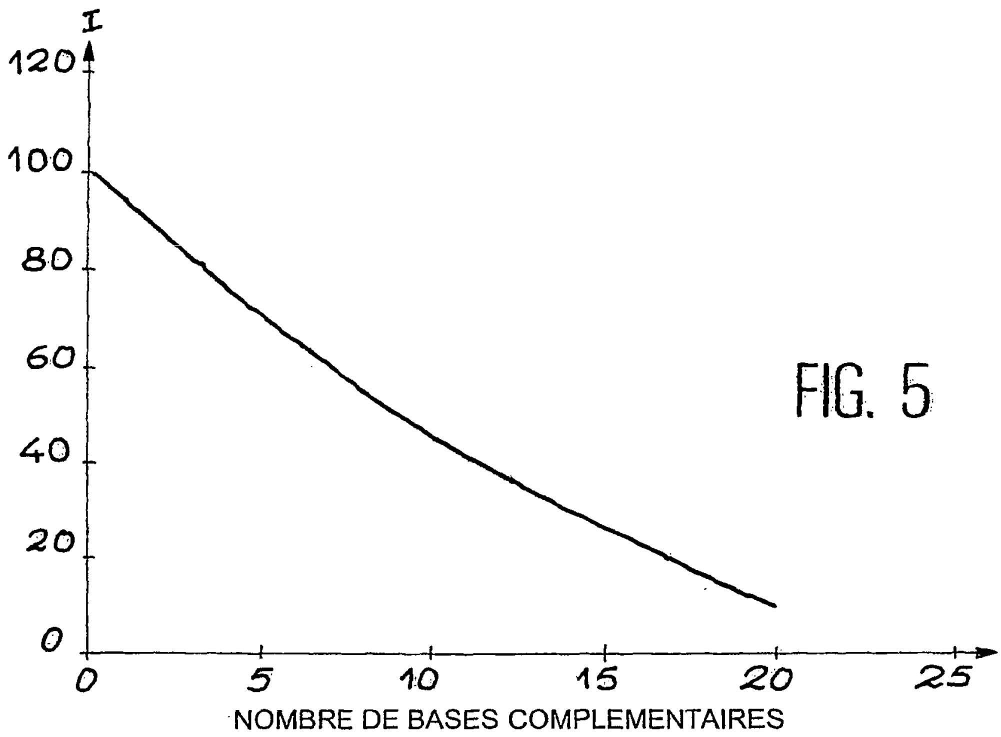
I
120
100
80
60
40
20
0
0
5
10
15
20
25
NOMBRE DE BASES COMPLEMENTAIRES

FIG. 5

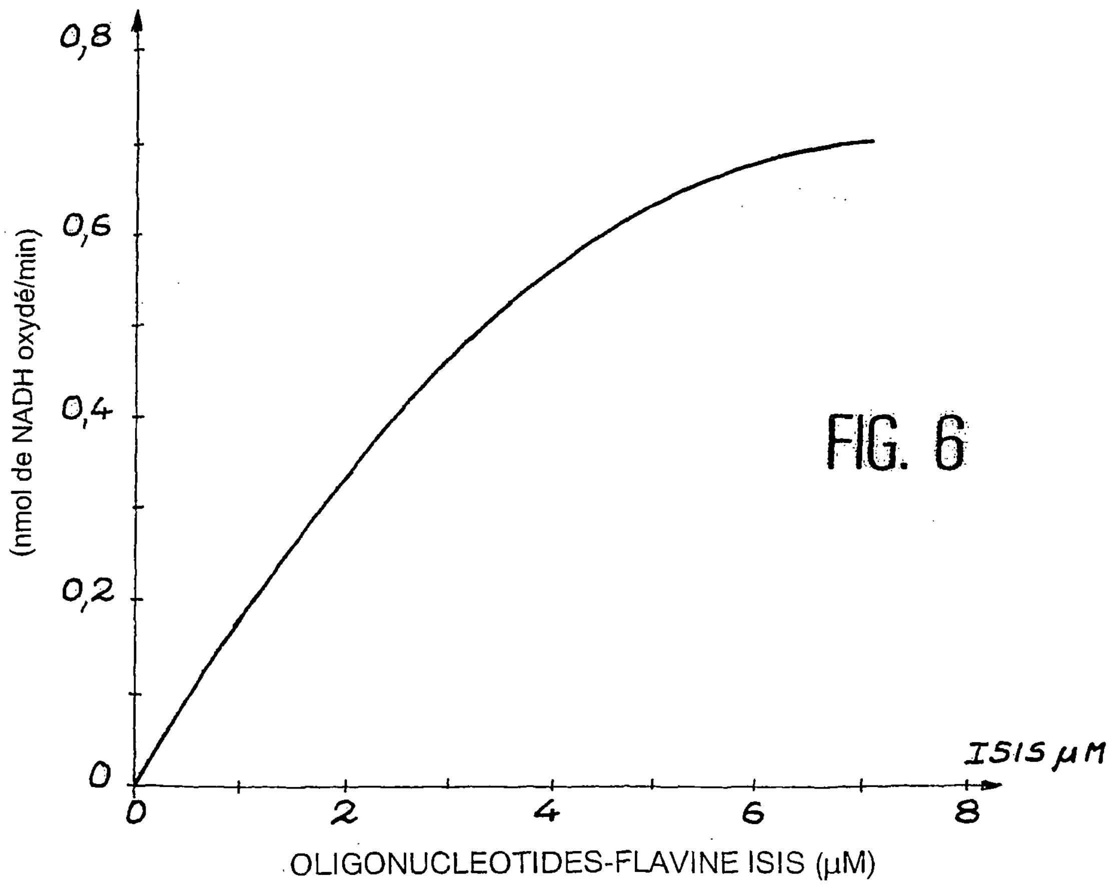
0,8
0,6
0,4
0,2
0
(nmol de NADH oxydé/min)
ISIS µM
0
2
4
6
8
OLIGONUCLEOTIDES-FLAVINE ISIS (µM)

FIG. 6

LISTE DE SEQUENCES

<110> COMMISSARIAT A L'ENERGIE ATOMIQUE UNIVERSITE JOSEPH FOURIER

<120> PROCEDE ET SUPPORT D'ANALYSE BIOLOGIQUE UTILISANT DES OLIGONUCLEOTIDES COMPORTANT UN MARQUEUR ACTIVABLE ENZYMATIQUEMENT

<130> B13836.3MDT

<140>
<141>

<150> FR N°0109460
<151> 2001-07-16

<160> 5

<170> PatentIn Ver. 2.1

<210> 1
<211> 20
<212> ADN
<213> Séquence artificielle

<220>
<223> Description de la séquence artificielle: séquence synthétique

<400> 1
ctctcccctt caccaccccc 20

<210> 2
<211> 42
<212> ADN

<213> Séquence artificielle

<220>
<223> Description de la séquence artificielle: séquence synthétique

<400> 2
gcctgatgag aggggaagtg gtgggggaga catagcccca cc 42

<210> 3
<211> 42
<212> ADN
<213> Séquence artificielle

<220>
<223> Description de la séquence artificielle: séquence synthétique

<400> 3
gcctgatgag aggggaagtg gtggcccaga catagcccca cc 42

<210> 4
<211> 42
<212> ADN
<213> Séquence artificielle

<220>
<223> Description de la séquence artificielle: séquence synthétique

<400> 4
gcctgatgag aggggaagtg gattcccaga catagcccca cc 42

<210> 5
<211> 45
<212> ADN
<213> Séquence artificielle

<220>
<223> Description de la séquence artificielle: séquence synthétique

<400> 5
ctcatcgtgt aaaaaaaaaa aggcagtact ggaagggcta attct 45